# EUROPEAN PATENT SPECIFICATION

(11) **EP 1 404 640 B1**
(45) Date of publication and mention of the grant of the patent: **12.09.2007**
(21) Application number: 02746222.5
(22) Date of filing: 05.07.2002
(51) Int. Cl.: C07C 69/90, C08G 63/08, A61K 31/235, A61P 29/00

(54) **HYDROXYBUTYRIC ACID ESTERS OF ACETYLSALICYLIC ACID, THEIR METHODS OF PRODUCTION AND APPLICATION AS WELL AS DRUGS CONTAINING THEM**
HYDROXYBUTTERSÄUREESTER VON ACETYLSALIZYLSÄURE, DEREN HERSTELLUNG UND ANWENDUNG SOWIE DIESE ENTHALTENDEN ARZNEIMITTEL
ESTERS D'ACIDE HYDROXYBUTYRIQUE AVEC L'ACIDE ACETLYSALICYLIQUE, LEURS PROCEDES DE PRODUCTION ET LEUR UTILISATION AINSI QUE DES MEDICAMENTS EN COMPORTANT

(30) Priority: 06.07.2001 PL 34848701
(43) Date of publication of application: 07.04.2004
(73) Proprietor: Centrum Chemii Polimer W Polskiej Akademii Nauk, 41-800 Zabrze (PL)
(72) Inventor: JEDLINSKI, Zbigniew, PL-44-100 Gliwice (PL); LUSZYK-JUZWA, Maria, PL-41-800 Zabrze (PL); KUREK, Anna, PL-44-100 Gliwice (PL); ZAWIDLAK, Barbara, PL-41-908 Bytom (PL)
(74) Representative: Hoffmeister, Helmut
(86) International application number: PCT/PL2002/000045
(87) International publication number: WO 2003/004451

(56) References cited:
- DE-A- 2 320 945
- US-A- 4 851 426

## Description

The objects of the present invention are new esters of acetylsalicylic acid with oligomers of 3-hydroxybutyric acid having the formula 1, methods of their production and application in medical treatment as well as drug containing them.

Acetylsalicylic acid (Aspirin) belongs to the group of non-steroid anti-inflammatory, drugs being at the same time effective in preventing myocardial ischemia and stroke. It also inhibits the aggregation of thrombocytes preventing the blood clotting. Although acetylsalicylic acid is a non-toxic drug, it irritates the mucous membrane of the stomach. Therefore the administration of acetylsalicylic acid is limited in the case of patients suffering from digestive tract illnesses.

Synthetic oligomers of 3-hydroxybutyric acid are analogues of natural poly([R]-3-hydroxybutyric) acid (PHB), which can be found in living organisms, for example in some cell membranes. The studies of oligomers of [R]-3-hydroxybutyric acid described earlier (Z. Jedliński, P. Kurcok, R. W. Lenz, Macromolecules 1998, 31, 6718; Z. Jedliński, M. Kowalczuk, P. Kurcok, patent RP 172412, 1993) as well as their applications in preparing synthetic cell membranes (S. Das, P. Kurcok, Z. Jedliński, R.N Reusch, Macromolecules, 1999, 32, 8781) showed that the oligomers of synthetic [R]-3-hydroxybutyric acid are hydrolyzed by enzymes in human body. They are completely biocompatible and their metabolites are non-toxic.

Esters of oligomers of synthetic 3-hydroxybutyric acid and acetylsalicylic acid have not been known so far.

The German laid open Patent Application 23 20 945 discloses esters of acetylsalicylic acid, in which the ester bond carries an n-acylic chain being substituted at is distant end by a carbalcoxy-residue. Furthermore the DE 23 20 945 A describes estrification reactions as methods for obtaining these esters.

Nearly same esters and methods as disclosed with the German laid open Patent Application 23 20 945 are known from the US-Patent No. 4,851,426.

Therefore the objects of the German laid open Patent Application 23 20 945 and the US-Patent No. 4,851,426 are esters of acetylsalicylic acid and methods of obtaining these known chemical structures employing estrification reactions.

In difference to the German laid open Patent Application 23 20 945 and the US-Patent No. 4,851,426 the present patent application discloses polymer-drug conjugates and novel methods for obtaining these polymer-drug conjugates based on the anionic ring-opening polymerization of β-butyrolactone in a highly polar aprotic solvent such as dimethyl sulfoxide (DMSO).

Novel esters of acetylsalicylic acid with oligomers of 3-hydroxybutyric acid according to the invention are presented in formula 1, where n equals from 2 to 25.

The method of obtaining compounds with formula **1**, where n equals from 2 to 25, according to the invention, consists in converting acetyisaiicyiic acid formula **2,** preferably with the use of sodium or potassium hydride or hydroxide, into monosodium or monopotassium salt formula **3**, where X denotes sodium or potassium, which in turn is reacted with β-butyrolactone causing the opening of β-butyrolactone ring at the carbon-oxygen position, the formation of an ester bond with acetylsalicylic acid if the lactone is present in excess, polymerization of the lactone take place.

The new compound obtained in this way is acetylsalicylic acid connected by ester bond with biocompatible polymer carrier being oligomer of 3-hydroxybutyric acid with 2 - 25 mers.
The bond between acetylsalicylic acid and biocompatible polymer carrier is easily hydrolyzed in living tissue.

According to the invention the drug containing known, pharmaceutically permissible carriers and acetylsalicylic acid as an active substance features acetylsalicylic acid connected by ester bond with oligomer of [R]-3-hydroxybutyric acid having from 2 to 25 mers and being biocompatible polymer carrier.

Because of their lypophylicity and affinity to cell membranes, oligomers of 3-hydroxybutyric acid combined with acetylsalicylic acid via ester bonds result in new pharmaceutical properties, particularly the irritation of mucous membrane of the digestive tract by acetylsalicylic acid is eliminated. The polymer carrier hydrolyzing gradually in the digestive tract facilitates permeation of the drug through cell membranes because of its lypophylicity and gradually releases the active agent, acetylsalicylic acid. According to the invention, the new drug-conjugates limit the necessity of using gelatin capsules to coat drugs containing acetylsalicylic acid.

The advantage of such a solution, according to the invention, is limiting the irritation of stomach caused by acetylsalicylic acid and release of the drug proceeds only in small intestine, which broadens the range of drug application in the form of a conjugate to the group of patients, who do not tolerate drugs containing acetylsalicylic acid. It has been found that, the gradual hydrolysis of oligo(3-hydroxybutyrate) and acetylsalicylic acid- polymer ester bond cleavage releases 3-hydroxybutyric acid and its oligomers, which are non-toxic and belong to the group of ketone bodies. According to the present state of arts ketone bodies are particularly useful in the treatment of patients suffering also from Alzheimer's disease because 3-hydroxybutyrate prevents neurons.

The conjugates can be used also in the form of tablets obtained by pressing after granulating medical substance with auxiliary substances like starch, corn cellulose, etc.

The methods of producing esters of acetylsalicylic acid with oligomers of [R]-3-hydroxybutyric acid (polymer-drug conjugates) according to the invention are as follows:

### Example I

### Synthesis of sodium salt of acetylsalicylic acid

In order to purify sodium hydride (NaH) used in the syntheses, it was washed with distilled tetrahydrofuran (THF) in atmosphere of neutral gas (argon) and then placed in a glass reactor in the quantity of 0.0392 g (0.0016 mole). Then 0.309 g (0.0017 mole) of acetylsalicylic acid was introduced into the reactor and then 5 ml of dimethyl sulfoxide (DMSO), previously dried over anhydrous calcium sulfate and stored over molecular sieves A4, were added. The content of the reactor was mixed with the use of magnetic stirrer for 2 hours in the atmosphere of nitrogen.

### Reaction of sodium acetylsalicylate obtained at the first stage with β-butyrolactone

[S]-β-butyrolactone was dried over calcium hydride (CaH₂) and distilled over metallic sodium. Then 0.41g (0.048 mole) of purified [S]- β-butyrolactone was introduced into the reactor containing sodium acetylsalicylate and the content was mixed by a magnetic stirrer in the atmosphere of argon until lactone reacted completely. Conversion was determined with the use of infrared spectroscopic analysis (IR). IR band at 1831 cm⁻¹ characteristic for carbonyl carbon of β-butyrolactone ester group disappeared, the band at 1735 cm⁻¹ characteristic for carbonyl carbon of ester groups in polyester was observed. After that 20 ml of acidified chloroform were introduced into the reactor and the obtained product was extracted by the mixture of chloroform-dimethyl sulfoxide-water. After the separation of diphase system, the product present in chloroform phase was precipitated in hexane and dried over low pressure.

### Analysis of the product:

Molecular weight (average) determined by GPC method (using polistyrene standards) Mₙ=451 [g/mole]
Elementary analysis C= 57.33 % H= 5.81 %
Analysis of the end product using mass spectrometry method ESI-MS and the analysis of positive ions showed the presence of following molecular ions:
m/z = 461 adequate to [C₉H₈O₄(C₄H₆O₂)₃HNa]⁺
m/z = 571 adequate to [C₉H₈O₄(C₄H₆O₂)₄HNa]⁺

### Example II

The synthesis was carried out in the same way as in Example I. 1.241 g (0.0517 mole) Of sodium hydride (NaH) (or 0.0103 mole of potassium hydride), 9.349 g (0.0519 mole) of acetylsalicylic acid and 31.100 g (0.3616 mole) of β-butyrolactone were used in the reaction.

### Analysis of the product

Molecular weight (average) determined by GPC method (using polystyrene standards) Mₙ=742 [g/mole]
Elementary analysis C = 56.71 % H= 6.28%
IR the band at 1735 cm⁻¹ characteristic for carbonyl carbon ester groups in polyester was observed.
Analysis of the end groups using mass spectrometry method ESI-MS and the analysis of positive ions showed the presence of following molecular ions:
m/z = 719 adequate to [C₉H₈O₄(C₄H₆O₂)₆HNa]⁺
m/z = 805 adequate to [C₉H₈O₄(C₄H₆O₂)₇HNa]⁺
m/z = 891 adequate to [C₉H₈O₄(C₄H₆O₂)₈HNa]⁺

### Example III

### Synthesis of potassium acetylsalicylate

3.26 g (0.018 mole) of acetylsalicylic acid and 30 ml of dimethyl sulfoxide (DMSO) previously dried over anhydrous calcium sulfate and stored over molecular sieves were introduced into the reactor. Then 0.92 g (0.017 mole) of potassium hydroxide was added. The content of the reactor was mixed with the use of a magnetic stirrer in the atmosphere of argon under IR control.

### Reaction of potassium acetylsalicylate obtained with [S]-β-butyrolactone

5.85 g (0.068 mole) of purified [S]-β-butyrolactone was introduced into the reactor containing potassium acetylsalicylate and the content was being mixed with a magnetic stirrer in the atmosphere of argon until lactone reacted completely. Conversion was determined by infrared spectroscopic analysis (IR). IR the band at 1830 cm⁻¹ characteristic for carbonyl carbon of β-butyrolactone ester group and appearance of the band at 1735 cm⁻¹ characteristic for carbonyl carbon ester groups in polyester were observed. After that 20 ml of acidified chloroform was introduced into the reactor and the obtained product was extracted by the mixture chloroform-dimethyl sulfoxide-water. After the separation of diphase system, the product present in chloroform phase was precipitated in hexane and dried over lowered pressure.

### Analysis of the product:

Molecular weight (average) determined by GPC method (using polystyrene standards) Mₙ=513 [g/mole]
Elementary analysis C = 57.36 % H= 5.95%
Analysis of the end groups using mass spectrometry method ESI-MS and the analysis of positive ions showed the presence of following molecular ions:
m/z = 461 adequate to [C₉H₈O₄(C₄H₆O₂)₃HNa]⁺
m/z = 547 adequate to [C₉H₈O₄(C₄H₆O₂)₄HNa]⁺

The formed conjugates contain chains of oligomeric 3-hydroxybutyric acid of various lengths depending on stoichiometric ratios of β-butyrolactone and sodium or potassium acetylsalicylate used in the reactions.

### Example IV

### Preparation of tablets containing a conjugate

A conjugate was mixed with dried starch, granulated and then stearic acid was added.
Amounts of components:

| | |
|---|---|
| Conjugate | 24.00 g (contains 10 g of acetylsalicylic acid) |
| Corn starch | 1.00 g |
| Stearic acid | 1.00 g |

Finally after mixing in the tablet machine tablets with the weight of 260 mg are formed (with the use of 9 mm stamps).

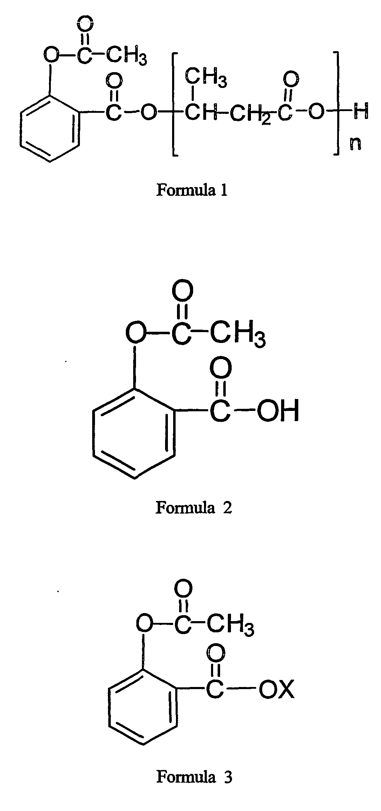

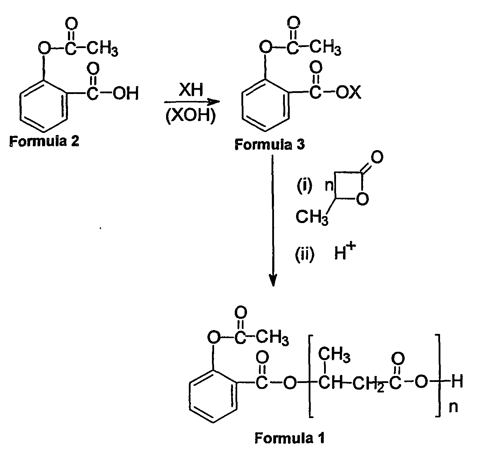

## Claims

1. Esters of acetylsalicylic acid with oligomers of 3-hydroxybutyric acid having the following formula: 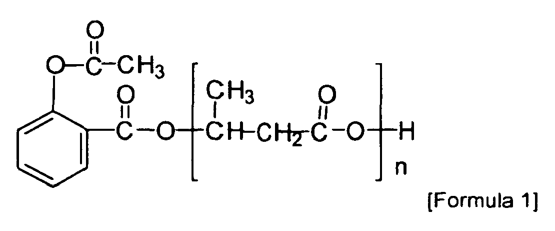 where n = 2 to 25

2. Method of obtaining esters according to claim 1, comprising the following steps:
a) converting acetylsalicylic acid having the following formula 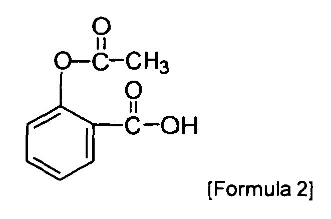 preferably in the presence of sodium or potassium hydrides or hydroxides, into monosodium or monopotassium salts having the following formula: 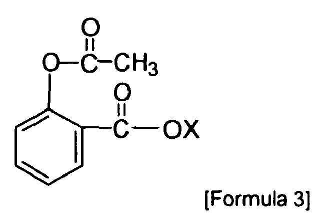 where X denotes sodium or potassium.
b) reacting the latter with β-butyrolacetone in dimethyl sufoxide (DMSO), causing the opening of lactone ring via the carbon-oxygen bond cleavage and a subsequent polymerization of the lactone, thus resulting in an ester according to claim 1.

3. A pharmaceutical product comprising an ester according to claim 1.

4. A pharmaceutical product comprising an ester according to claim 3,
**characterized by** the fact that it contains additives selected from the group consisting of corn starch, cellulose, stearic acid, copolymer of metacrylic acid and other pharmaceutically permissible auxiliary substances.

5. Use of an ester according to claim 1 or a pharmaceutical product according to claims 3 or 4 for the manufacture of a treatment against inflammations, myocardial ischemia and stroke, aggregation of thrombocytes, blood clotting and Alzheimer's disease.

## Patentansprüche

1. Ester der Acetylsalicylsäure mit Oligomeren der 3-Hydroxybuttersäure, die folgende Formel haben: 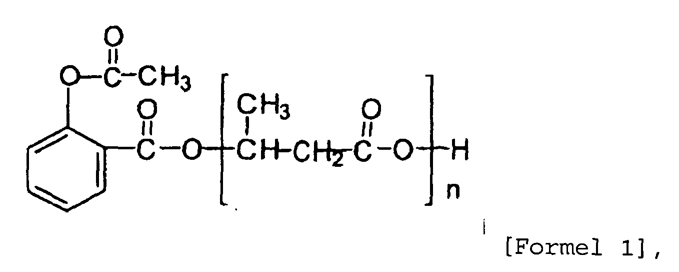 worin n = 2 bis 25.

2. Verfahren zur Erzeugung von Estern gemäß Anspruch 1, umfassend folgende Schritte:
a) Umwandlung der Acetylsalicylsäure, die folgende Formel hat: 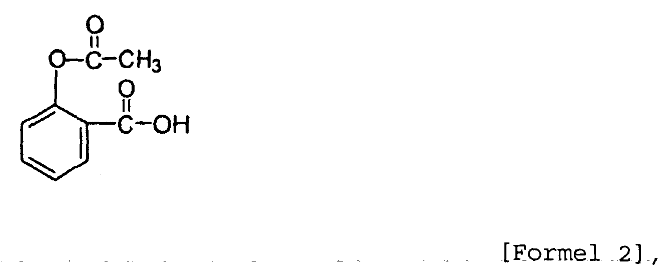 vorzugsweise in Gegenwart von Natrium- oder Kaliumhydriden oder -hydroxiden, in Mono-Natrium- oder Mono-Kalium-Salze mit folgenden Formeln: 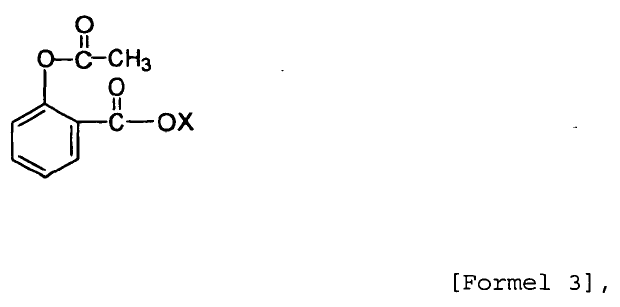 wobei X Natrium oder Kalium bezeichnet;
b) Reagierenlassen der genannten Salze mit β-Butyrolacton in Dimethylsulfoxid (DMSO), wobei die Öffnung des Lactonrings durch Aufspaltung der Kohlenstoff-Sauerstoff-Bindung und eine nachfolgende Polymerisierung des Lactons erzielt wird, wobei sich ein Ester gemäß Anspruch 1 ergibt.

3. Pharmazeutisches Produkt, einen Ester gemäß Anspruch 1 enthaltend.

4. Pharmazeutisches Produkt enthaltend einen Ester gemäß Anspruch 3, **dadurch gekennzeichnet, dass** es Additive, ausgewählt aus der Gruppe Maisstärke, Cellulose, Stearinsäure, Copolymere von Metacrylsäure und anderen pharmazeutisch zugelassenen Hilfssubstanzen, enthält.

5. Verwendung eines Esters gemäß Anspruch 1 oder eines pharmazeutischen Produktes gemäß Anspruch 3 oder 4 zur Herstellung eines Medikamentes gegen Entzündungen, Myokard-Ischämie und Schlaganfall, Aggregation von Thrombozyten, Blutgerinnung und Alzheimer'sche Krankheit.

## Revendications

1. Esters d'acide acétylsalicylique et d'oligomères d'acide 3-hydroxybutyrique répondant à la formule : 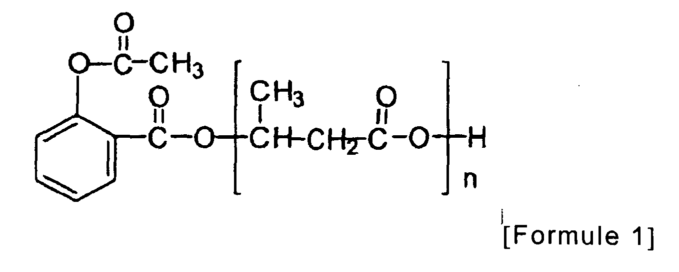 dans laquelle n est compris entre 2 et 25.

2. Procédé de préparation d'esters suivant la revendication 1, comportant les étapes ci-après :
a) Conversion de l'acide acétylsalicylique de formule : 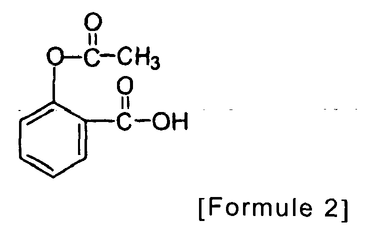 de préférence en présence d'hydrures ou hydroxydes de sodium ou potassium en sels monosodiques ou monopotassiques répondant à la formule : 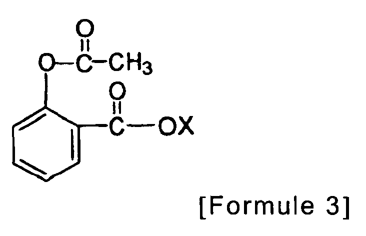 dans laquelle X désigne le sodium ou le potassium.
b) Réaction du produit obtenu avec de la β-butyrolactone dans le diméthyl sufoxide (DMSO), de manière à ouvrir le noyau lactone par scission de la liaison carbone-oxygène et polymérisation subséquente de la lactone, ce qui conduit à un ester suivant la revendication 1.

3. Produit pharmaceutique comportant un ester suivant la revendication 1.

4. Produit pharmaceutique comportant un ester suivant la revendication 3, **caractérisé en ce qu'**il contient des additifs choisis dans le groupe comprenant l'amidon de maïs, la cellulose, l'acide stéarique, le copolymère d'acide méthacrylique et autres substances secondaires pharmaceutiquement acceptables.

5. Utilisation d'un ester suivant la revendication 1 ou d'un produit pharmaceutique suivant la revendication 3 ou 4 pour la fabrication d'un agent de traitement contre les symptômes inflammatoires, les ischémies du myocarde et attaques cardiaques, l'aggrégation des thrombocytes, la coagulation sanguine et la maladie d'Alzheimer.
